(19) Europäisches Patentamt
European Patent Office
Office européen des brevets

(11) Veröffentlichungsnummer: **0 042 098**
**A2**

# (12) EUROPÄISCHE PATENTANMELDUNG

(21) Anmeldenummer: **81104213.4**

(22) Anmeldetag: **02.06.81**

(51) Int. Cl.³: **C 07 C 67/055,** C 07 C 69/16, B 01 J 27/08

(30) Priorität: **18.06.80 DE 3022722**

(43) Veröffentlichungstag der Anmeldung: **23.12.81**
**Patentblatt 81/51**

(84) Benannte Vertragsstaaten: **BE CH DE FR GB IT LI**

(71) Anmelder: **BASF Aktiengesellschaft,**
**Carl-Bosch-Strasse 38, D-6700 Ludwigshafen (DE)**

(72) Erfinder: **Fischer, Rolf, Dr., Bergstrasse 98,**
**D-6900 Heidelberg (DE)**
Erfinder: **Weitz, Hans-Martin, Dr., Auf dem Koeppel 40,**
**D-6702 Bad Duerkheim (DE)**

## (54) Verfahren zur Herstellung von 1-Buten-3,4-diolmonoestern und neue 1-Buten-3-alkyl-3,4-diolmonoester.

(57) Verfahren zur Herstellung von 1-Buten-3,4-diolmonoestern durch Umsetzung von 1,3-Butadienen in Gegenwart von Palladium, Platin oder Salzen dieser Metalle mit Sauerstoff und Carbonsäuren in Gegenwart von Bromverbindungen und neue 1-Buten-3,4-diolmonoester der Formel

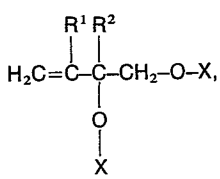

in der einer der Reste X ein Wasserstoffatom, der andere Rest X einen $R_3$–CO–Rest, $R^1$ ein Wasserstoffatom oder einen Alkylrest mit 1 bis 3 C-Atomen und die Reste $R^2$ und $R^3$ Alkylreste mit 1 bis 3 C-Atomen bedeuten.

EP 0 042 098 A2

Verfahren zur Herstellung von 1-Buten-3,4-diolmonoestern und neue 1-Buten-3-alkyl-3,4-diolmonoester

Diese Erfindung betrifft ein Verfahren zur Herstellung von 1-Buten-3,4-diolmonoestern durch Umsetzung von 1,3-Butadienen mit Sauerstoff und Carbonsäuren in Gegenwart von Palladium, Platin oder Salzen dieser Metalle und von Bromverbindungen sowie neue 1-Buten-3-alkyl-3,4-diolmonoester.

In der DE-PS 2 217 452 wird ein Verfahren zur Herstellung von Diacetoxibutenen durch Umsetzung von Butadien mit Sauerstoff und Essigsäure ("Acetoxilierung") beschrieben, bei dem man Palladium-Trägerkatalysatoren verwendet, die zusätzlich Antimon, Wismut, Tellur oder Selen enthalten. Die in der DE-OS 2 728 574 beschriebene Acetoxilierung führt man in Gegenwart von schwefelhaltigen Palladium-Trägerkatalysatoren durch, die außerdem noch andere Elemente enthalten können. Nach den Angaben der DE-OS 2 820 519 verwendet man Trägerkatalysatoren, die Palladium und Kupfer in Form einer intermetallischen Verbindung enthalten. Die DE-OS 2 417 558 beschreibt für Acetoxilierungen geeignete Platin-Trägerkatalysatoren, die zusätzlich eines der Elemente Phosphor, Arsen, Antimon, Selen oder Tellur enthalten. Acetoxiliert man Butadien nach diesen bekannten Verfahren, so entstehen überwiegend cis- und trans-2-Buten-1,4-dioldiacetate. 1-Buten-3,4-diolmono- und 1-Buten-3,4-dioldiacetate werden dagegen nur in untergeordnetem Maße gebildet.

Acetoxiliert man Butadien nach dem Verfahren der DE-PS 2 012 903, bei dem metallisches Palladium enthaltende Katalysatoren verwendet werden, bei deren Herstellung Kupfersalze, wie Kupferchlorid oder Kupferacetat

Hee/BL

COMPLETE DOCUMENT

zugegeben werden, so überwiegt ebenfalls die Bildung von 2-Buten-1,4-dioldiacetaten.

Nach den Angaben der GB-PS 1 138 366 erhält man durch Umsetzung von Piperylen mit Luft in Gegenwart von in Eisessig gelöstem Palladiumacetat, Kupferacetat und Lithiumacetat Gemische aus 1,4-Diacetoxy-2-penten und 4,5-Diacetoxy-2-penten (Molverhältnis 4:1). In ähnlicher Weise wird Butadien nach dem Verfahren der GB-PS 1 277 837 mit Sauerstoff in Gegenwart von in Eisessig gelöstem Palladiumacetat, Kupferacetat und Kaliumacetat zu 1,4-Diacetoxy--2-buten umgesetzt.

Acetoxiliert man Butadien in Gegenwart von metallisches Palladium enthaltenden festen Katalysatoren mit Aktivkohle als Träger, die in Lösungen von Kupferchlorid, Lithiumacetat und Antimontrichlorid in Essigsäure und Acetanhydrid (japanische Patentanmeldung 39 004 (1972)) oder von Kupferchlorid in Essigsäure und Acetanhydrid (japanische Patentanmeldung 39 003 (1972)) suspendiert werden, so besteht das Reaktionsprodukt zu 73,4 % bzw. 59,3 % aus 1-Buten-3,4-diol-diacetat, 0,4 bzw. 2,9 % aus 1-Buten-3,4-diol-monoacetat und 26,2 % bzw. 37,8 % aus 2-Buten-1,4-dioldiacetaten.

Bei diesen bekannten Verfahren der Acetoxilierung von Butadienen werden somit als Hauptprodukte 1-Buten-3,4-dioldiacetate oder 2-Buten-1,4-dioldiacetate gebildet. Es wurde nun überraschenderweise gefunden, daß man bei der Umsetzung von 1,3-Butadienen in Gegenwart von Palladium, Platin oder Salzen dieser Metalle mit Sauerstoff und Carbonsäuren überwiegend 1-Buten-3,4-diolmonoester erhält, wenn man die Umsetzung in Gegenwart von Bromverbindungen durchführt.

Als 1,3-Butadiene sind außer Butadien auch andere konjugierte Diolefine, wie Isopren, 2,3-Dimethyl-1,3-butadien, 2-Äthyl-1,3-butadien, 2-Propyl-1,3-butadien, 1,3-Pentadiene, wie Piperylen oder durch Acyloxygruppen substituierte 1,3-Butadiene, wie 1-Acetoxybutadien, 1-Acetoxy-2-methyl-1,3-butadien oder 1-Acetoxy-3-methyl-1,3-butadien verwendbar. Die genannten Diolefine können für sich oder als Mischungen, die z.B. auch noch andere Kohlenwasserstoffe, wie Monoolefine und Paraffinkohlenwasserstoffe enthalten, eingesetzt werden. Solche Mischungen stehen z.B. als sogenannte $C_4$-Schnitte zur Verfügung. Als Carbonsäuren kommen niedermolekulare Carbonsäuren mit 1 bis 3 C-Atomen, wie Ameisensäure, Essigsäure oder Propionsäure in Betracht.

Als Katalysatoren verwendet man Palladium, Platin oder Salze dieser Metalle, wobei diese Katalysatoren noch andere aktive Bestandteile enthalten können. Beispielsweise geeignet sind Trägerkatalysatoren, die als aktive Bestandteile, welche auf dem Trägermaterial aufgebracht sind, Palladium oder Platin und Kupfer und/oder Tellur enthalten.

Die für das erfindungsgemäße Verfahren geeigneten Katalysatoren lassen sich in bekannter Weise, z.B. nach den Angaben der DE-PS 2 217 452, DE-OS 2 820 519 oder DE-OS 2 417 558 herstellen. Katalysatoren der genannten Art enthalten beispielsweise, bezogen auf das Katalysatorgewicht, 1 bis 10 % Palladium oder Platin, 0,1 bis 30 % Kupfer und/oder 0,01 bis 10 % Tellur. Bevorzugt ist die Verwendung eines Trägerkatalysators, der je Grammatom Palladium oder Platin 0,01 bis 6, vorzugsweise 1 bis 3,5 Grammatome Kupfer und/oder 0,01 bis 1, vorzugsweise 0,01 bis 0,4 Grammatome Tellur enthält.

Die Gesamtmenge der auf den Träger aufgebrachten katalytisch wirksamen Metalle beträgt, bezogen auf den Trägerkatalysator, zweckmäßig z.B. 0,01 bis 30 Gew.-%. Größere und kleinere Konzentrationen sind allerdings auch möglich.

Als Trägermaterial enthalten die Katalysatoren z.B. Aktivkohle, Bauxit, Bimsstein, Kieselgel, Kieselgur oder andere Formen der Kieselsäure, Magnesia, Ton oder Tonerde.

Man kann die katalytisch wirksamen Metalle z.B. auch ohne Trägermaterial anwenden, indem man sie in Form von Salzen im Reaktionsgemisch löst oder suspendiert.

Als erfindungsgemäß zu verwendende Bromverbindungen kommen z.B. Metallbromide, Bromwasserstoffsäure oder andere Bromverbindungen in Betracht, die unter den Umsetzungsbedingungen Bromidionen freisetzen. Als Metallbromide können z.B. solche der Elemente der 1. bis 6. Hauptgruppe, der 1. bis 8. Nebengruppe und der Lanthaniden- und Aktinidenelemente eingesetzt werden. Andere Verbindungen die unter den Reaktionsbedingungen Bromidionen abzuspalten vermögen, sind z.B. die Hydrobromide organischer Basen wie Aminen. Die bromhaltigen Verbindungen werden im Reaktionsmedium, also im allgemeinen in der Carbonsäure gelöst oder bei zu geringer Löslichkeit suspendiert. Pro Grammäquivalent Palladium oder Platin werden im allgemeinen 0,1 bis 10 Grammäquivalente Bromidionen zugesetzt.

Die Umsetzung zur Herstellung der 1-Buten-3,4-diolmonoacetate nimmt man in an sich bekannter Weise in der Gas- oder Flüssigphase bei Temperaturen von 70 bis 180°C vor. In der Gasphase arbeitet man vorzugsweise bei 120 bis 150°C und in der Flüssigphase vorzugsweise bei 70 bis 110°C. Der Reaktionsdruck ist durch die Verfahrensweise gegeben und kann zwischen atmosphärischem Druck und z.B. 100 bar

betragen. Das Verfahren kann chargenweise oder kontinuierlich z.B. mit fixiertem Bett, Wirbelbett oder Dreiphasenfließbett durchgeführt werden.

Von den nach dem erfindungsgemäßen Verfahren erhältlichen 1-Buten-3,4-diolmonoestern sind die Verbindungen der Formel

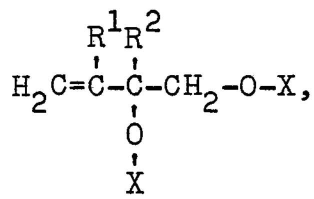

in der einer der Reste X ein Wasserstoffatom, der andere Rest X einen $R_3$-CO-Rest, $R^1$ ein Wasserstoffatom oder einen Alkylrest mit 1 bis 3 C-Atomen und die Reste $R^2$ und $R^3$ Alkylreste mit 1 bis 3 C-Atomen bedeuten, neu.

Von diesen neuen 1-Buten-3,4-diolmonoestern sind die Verbindungen der Formel

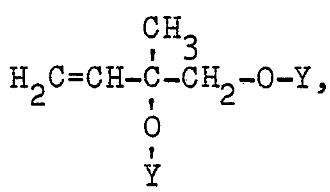

in der einer der Reste Y ein Wasserstoffatom und der andere Rest Y einen $H_3C$-CO-Rest bedeuten, von besonderem technischem Interesse.

Die nach dem Verfahren der Erfindung herstellbaren 1-Buten-3,4-diolmonoester, in denen im allgemeinen die 3-Hydroxy-4-acyloxy-1-butene überwiegen, stellen wertvolle Zwischenprodukte dar. Da eine der beiden Hydroxylgruppen des

zugrundeliegenden 1-Buten-3,4-diols verestert ist, läßt sich die zweite Hydroxylgruppe selektiv, z.B. durch Oxidation zur Aldehyd- oder Carboxylgruppe, abwandeln. 3-Acetoxy-4-hydroxy- und 3-Hydroxy-4-acetoxy-1-buten können ähnlich wie das 3,4-Diacetoxy-1-buten (s. Pure and Applied Chemistry <u>43</u>, 543 (1975)) für die Herstellung von 2-Methyl-4-acetoxy-2-butenal (4-Acetoxytiglinaldehyd), einem für die Synthese vom Vitamin A geeigneten $C_5$-Baustein, verwendet werden. Das bei der Acetoxilierung von Isopren und anschließender Verseifung der Isomeren 3,4-Mono- und 3,4-Diacetate anfallende 3-Methyl-3,4-dihydroxy-1-buten läßt sich zu Vinylmilchsäure, dem Baustein für ein wichtiges Fungizid (s.DE-OS 2 207 576) oxidieren. 2-Methyl-3,4-dihydroxy-1-buten kann als Terpenbaustein Verwendung finden.

In den Beispielen werden folgende Abkürzungen benutzt:

| | | |
|---|---|---|
| trans-1,4-Diacetoxy-2-buten | = | trans-1,4-BEDA |
| cis-1,4-Diacetoxy-2-buten | = | cis-1,4-BEDA |
| 3,4-Diacetoxy-1-buten | = | 3,4-BEDA |
| 3,4-Hydroxyacetoxy-1-butene | = | 3,4-BEMA |
| 1,4-Hydroxiycetoxy-2-butene | = | 1,4-BEMA |

Bei den Prozentangaben handelt es sich um Gewichtsprozent.

<u>Beispiel 1</u>

a) Katalysatorherstellung

89,6 g Kupferpulver, gelöst in 660 $cm^3$ 33 %iger Salpetersäure werden bei Raumtemperatur mit 83,4 g $PdCl_2$, gelöst in 400 $cm^3$ eines warmen Gemisches aus 66 %iger Salpetersäure und 32 %iger Salzsäure (Volumenverhältnis 1:1) und 6,25 g $TeO_2$ in 1000 $cm^3$ warmer, 16 %iger Salzsäure, ver-

mischt. Man legt 1000 g Aktivkohle (0,3 bis 0,5 mm) vor, die zuvor bei 70°C 5 Stunden lang mit 15 %iger Salpetersäure gerührt, nach dem Abnutschen neutral gewaschen und bei 150°C unter Vakuum getrocknet worden war und fügt die vereinigte Metallsalzlösung hinzu. Anschließend gibt man soviel Wasser hinzu, daß die Kohle völlig benetzt ist. Dann wird am Rotationsverdampfer bei 85°C und Wasserstrahlvakuum zur Trockene eingedampft. Der Katalysator wird 2 Stunden bei 150°C im Vakuumtrockenschrank und dann 2 Stunden bei 150°C im Röhrenofen unter strömendem Stickstoff getrocknet. Anschließend leitet man zur Aktivierung des Katalysators bei 400°C bei Raumtemperatur mit Methanol gesättigten Stickstoff und schließlich 0,5 Stunden Wasserstoff (20 l/Stunde) bei 800°C über den Katalysator. Man läßt unter strömendem Stickstoff auf Raumtemperatur abkühlen.

Nach der Elementaranalyse enthält der Katalysator 5,14 % Palladium, 8,5 % Kupfer und 0,51 % Tellur. Die Röntgenanalyse zeigt eine $PdCu_3$-Phase, die praktisch kein PdCu enthält. Beugungslinien, die Pd-Te-Phasen entsprechen könnten, werden nicht beobachtet. Die Gitterkonstante der $PdCu_3$-Phase beträgt 3,693 Å. Die gemessenen d-Werte in nm (CuK$\alpha$-Strahler) sind in der nachfolgenden Tabelle angegeben.

| $PdCu_3$ | (hkl) |
|---|---|
| 0,37 | 001 |
| 0,26 | 011 |
| 0,213 | 111 |
| 0,1848 | 002 |
| 0,1305 | 022 |

b) Acetoxilierung von Butadien

Eine Apparatur, die aus einem 1-Liter-Dreihalskolben mit Anschützaufsatz, Tropftrichter, Begasungsrührer, Innenthermometer, Gaseinleitungsrohr und Rückflußkühler mit aufgesetztem Trockeneiskühler besteht, wird mit Stickstoff gespült. Dann gibt man eine Suspension von 25,9 des nach Beispiel 1a) hergestellten Katalysators in 543 g Eisessig, der 4 g Lithiumbromid gelöst enthält, in die Apparatur. Man erhitzt auf 95°C und leitet im Verlauf von 4 Stunden gleichzeitig je 12 l Sauerstoff und Butadien ein. Nach beendeter Zugabe wird bei 95°C 30 Minuten lang Stickstoff durch das Reaktionsgemisch geleitet. Man läßt abkühlen und entfernt den Katalysator durch Filtrieren. Die anfallende Essigsäurelösung (579 g) enthält nach der GC-Analyse 1,65 % trans-1,4-BEDA, 0,12 % cis-1,4-BEDA, 1,21 % 3,4-BEDA und 4,82 % 3,4-BEMA. Demnach sind insgesamt 44,1 g Butendioldiacetate und -monoacetate entstanden. Der Anteil an 3,4-BEMA beträgt 68,1 Mol-%, die Summe aus 3,4 BEMA und 3,4-BEDA 81,1 Mol-% (bezogen auf die Summe aller Mono- und Diacetate).

Zur Gewinnung von 3,4-BEMA wird das Reaktionsgemisch am Rotationsverdampfer bei 60°C und Wasserstrahlvakuum eingedampft. Der Rückstand wird mit Wasser aufgenommen. Die Wasserphase wird der Reihe nach je drei Mal mit Cyclohexan und Chloroform extrahiert. Die vereinigten Chloroformphasen werden eingedampft, der Rückstand wird fraktioniert destilliert. Hierbei erhält man eine Fraktion vom Siedepunkt 104 bis 106°C, 25 mbar, ($n_D^{20}$= 1,4417), die zu mehr als 91 % aus den beiden möglichen 1-Buten-3,4-diolmonoacetaten besteht. Durch $^1$H-NMR-Analyse in Gegenwart eines Europium-Shiftreagenzes läßt sich eine Isomerenverteilung von 77 % 3-Hydroxy-4-acetoxy-1-buten und 23 % 3-Acetoxy-4-hydroxi-1-buten feststellen.

Beispiel 2

Wird Beispiel 1b unter gleichen Bedingungen, aber in Abwesenheit von Lithiumbromid durchgeführt, so findet man in den 565 g der erhaltenen Essigsäurelösung 6,72 % trans-1,4-BEDA, 1,01 % cis-1,4-BEDA, 1,01 % 3,4-BEDA und nur 0,05 % 3,4-BEMA.

Beispiele 3 bis 9

Man verfährt wie im Beispiel 1b beschrieben, wobei man jedoch anstelle von Lithiumbromid die in der folgenden Tabelle genannten Halogenide bzw. 47 %ige Bromwasserstoffsäure verwendet. Die Ergebnisse sind aus der Tabelle ersichtlich. Beispiel 3 ist ein Vergleichsbeispiel, das die geringe Wirksamkeit von Chloriden zeigt.

Tabelle 1

| Beispiele | Bromidzusatz [Mol] | Rohaustrag [g] | 3,4-BEMA [Gew.-%] | trans-1,4-BEDA [Gew.-%] | cis-1,4-BEDA [Gew.-%] | 3,4-BEDA [Gew.-%] | BEDA + BEMA [g] | % 3,4-BEMA [Mol-%-bezogen auf BEDA + BEMA] |
|---|---|---|---|---|---|---|---|---|
| 3 | LiCl (0,046) | 564 | 1,41 | 2,47 | 0,28 | 0,81 | 28,0 | 34,4 |
| 4 | KBr (0,046) | 572 | 5,13 | 1,45 | 0,11 | 1,11 | 44,6 | 71,8 |
| 5 | $CaBr_2 \cdot 2H_2O$ (0,023) | 571 | 4,05 | 1,31 | 0,11 | 1,03 | 37,1 | 68,6 |
| 6 | $ZnBr_2$ (0,023) | 560 | 4,11 | 1,19 | 0,13 | 0,93 | 35,6 | 70,7 |
| 7 | $CoBr_2 \cdot 6H_2O$ (0,023) | 570 | 5,53 | 1,22 | 0,10 | 0,98 | 44,7 | 76,2 |
| 8 | $AlBr_3$ (0,015) | 562 | 4,72 | 1,19 | 0,11 | 0,99 | 39,5 | 73,2 |
| 9 | HBr (0,046) | 574 | 5,43 | 0,88 | 0,09 | 0,89 | 41,3 | 79,2 |

Beispiel 10

Man verfährt wie im Beispiel 1b beschrieben, wobei man jedoch einen analog Beispiel 1a hergestellten Palladium-Kupfer-Tellur-Katalysator (4,9 % Pd; 8,3 % Cu; 0,55 % Te) mit Aktivkohle als Träger und anstelle von Lithiumbromid 10,3 g Kupfer(II)- bromid verwendet. Man erhält 564 g einer Essigsäurelösung, die 1,94 % trans-1,4-BEDA, 0,17 % cis-1,4-BEDA, 1,39 % 3,4-BEDA und 4,77 % 3,4-BEMA enthält.

Beispiel 11 (Vergleichsbeispiel)

Wird Beispiel 10 unter gleichen Bedingungen, aber in Abwesenheit von Kupfer(II)bromid durchgeführt, so werden in der erhaltenen Essigsäurelösung (571 g) 7,96 % trans-1,4-BEDA, 1,35 % cis-1,4-BEDA, 1,21 % 3,4-BEDA und 0,05 % 3,4-BEMA gefunden.

Beispiel 12

Man verfährt wie im Beispiel 1b beschrieben, wobei man als Katalysator jedoch 25 g eines nach der DE-PS 2 217 452 hergestellten Palladium-Tellur-Katalysators (5,1 % Pd; 0,6 % Te), der Aktivkohle als Träger enthält, verwendet. In der erhaltenen Essigsäurelösung (556 g) werden 0,6 % trans-1,4-BEDA, 0,06 % cis-1,4-BEDA, 0,35 % 3,4-BEDA und 1,25 % 3,4-BEMA gefunden.

Beispiel 13

Man verfährt wie im Beispiel 1b beschrieben, wobei man als Katalysator jedoch 25 g eines nach der DE-OS 2 820 519 hergestellten Palladium-Kupferkatalysators (5,31 % Pd; 9,47 % Cu), der Aktivkohle als Träger enthält, verwendet. In der erhaltenen Essigsäurelösung (556 g) werden 0,29 %

trans-1,4-BEDA, 0,02 % cis-1,4-BEDA, 0,41 % 3,4-BEDA und 1,68 % 3,4-BEMA gefunden.

Beispiel 14

In der in Beispiel 1b beschriebenen Apparatur werden durch eine Lösung von 2,66 g Palladium(II)acetat, 7,04 g Kupfer-(II)acetat . $H_2O$ und 4 g Lithiumbromid in 543 g Eisessig im Verlauf von 4 Stunden gleichzeitig insgesamt je 12 l Sauerstoff und Butadien geleitet. Danach zeigt die GC-Analyse der Essigsäurelösung (558 g) einen Gehalt von 0,31 % trans-1,4-BEDA, 0,03 % cis-1,4-BEDA, 0,34 % 3,4-BEDA und 1,73 % 3,4-BEMA.

Beispiel 15 (Vergleichsbeispiel)

Wird das Lithiumbromid in Beispiel 14 durch 3,04 g Lithiumacetat ersetzt, und verfährt man im übrigen wie im Beispiel 14 beschrieben, so sind in dem Reaktionsaustrag (555 g) 2,46 % trans-1,4-BEDA, 0,26 % cis-1,4-BEDA, 0,41 % 3,4-BEDA und 0,06 % 3,4-BEMA enthalten.

Beispiel 16

In der in Beispiel 1b beschriebenen Apparatur werden 25 g des nach Beispiel 1a hergestellten Katalysators in 543 g Essigsäure suspendiert, die 4 g Lithiumbromid gelöst enthält. Man erhitzt auf 95°C und leitet im Verlauf von 4 Stunden 12 l Sauerstoff ein. Gleichzeitig werden 34 g Isopren zugetropft. Nach beendeter Zugabe werden während einer weiteren halben Stunde 1,5 l Sauerstoff eingeleitet. Dann wird wie im Beispiel 1b beschrieben aufgearbeitet. Die anfallende Essigsäurelösung (573 g) enthält laut GC-Analyse 7,92 % 2- und 3-Methyl-3-hydroxy(acetoxy)-4-acetoxy(hydroxy)-1-butene, 1,88 % 2- und 3-Methyl-3,4-

-diacetoxy-1-butene, 0,23 % cis-2-Methyl-1,4-diacetoxy-2-buten und 1,34 % trans-2-Methyl-1,4-diacetoxy-2-buten. Bei der fraktionierten Destillation der Essigsäurelösung werden 56,8 g Mono- und Diacetatgemisch vom Siedepunkt 59 bis 62°C/0,3 bis 0,5 mbar, $n_D^{20}$= 1,4425 erhalten. Durch $^1$H-NMR-Analyse mit Europium-Shiftreagenz konnte gezeigt werden, daß der Monoacetatanteil zu rund 70 % aus 2-Methyl-3-hydroxy(acetoxy)-4-acetoxy(hydroxy)-1-buten und zu 30 % aus 3-Methyl-3-hydroxy(acetoxy)-4-acetoxy(hydroxy)-1-buten bestand, in dem 3-Methyl-3-hydroxy-4-acetoxy-1-buten überwog.

Patentansprüche

1. Verfahren zur Herstellung von 1-Buten-3,4-diolmonoestern durch Umsetzung von 1,3-Butadienen in Gegenwart von Palladium, Platin oder Salzen dieser Metalle mit Sauerstoff und Carbonsäuren, <u>dadurch gekennzeichnet</u>, daß man die Umsetzung in Gegenwart von Bromverbindungen durchführt.

2. Verfahren nach Anspruch 1, <u>dadurch gekennzeichnet</u>, daß man als Bromverbindungen Metallbromide oder Bromwasserstoffsäure verwendet.

3. 1-Buten-3,4-diolmonoester der Formel

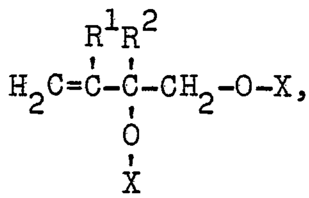

in der einer der Reste X ein Wasserstoffatom, der andere Rest X einen $R_3$-CO-Rest, $R^1$ ein Wasserstoffatom oder einen Alkylrest mit 1 bis 3 C-Atomen und die Reste $R^2$ und $R^3$ Alkylreste mit 1 bis 3 C-Atomen bedeuten.

4. 1-Buten-3,4-diolmonoester der Formel

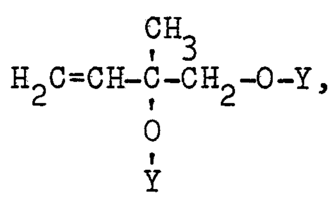

in der einer der Reste Y ein Wasserstoffatom und der andere Rest Y einen $H_3C$-CO-Rest bedeuten.